# FASCICULE DE BREVET EUROPEEN

(11) **EP 0 461 028 B1**
(45) Date de publication et mention de la délivrance du brevet: **01.09.1993**
(21) Numéro de dépôt: 91401464.2
(22) Date de dépôt: 05.06.1991
(51) Int. Cl.: A61B 6/00

(54) **Procédé de basculement avec un ostéodensimètre d'un examen antéro-postérieur à un examen latéral**
Kippverfahren für Knochendichtemesser einer vorhergehenden und nachfolgenden Untersuchung bei seitlicher Prüfung
Tilting process for osteodensitymeter for a leading and trailing examen of a lateral examen

(30) Priorité: 06.06.1990 FR 9006983
(43) Date de publication de la demande: 11.12.1991
(73) Titulaire: SOPHA MEDICAL, F-75008 Paris (FR)
(72) Inventeur: Pare, Christian, F-75116 Paris (FR); Bonnet, Guy, F-75116 Paris (FR); Fleury, Christophe, F-75116 Paris (FR)
(74) Mandataire: Whitten, George Alan

(56) Documents cités:
- EP-A- 0 253 742
- WO-A-86/07531
- WO-A-88/08688
- US-A- 3 639 764

## Description

La présente invention a pour objet un procédé de basculement, avec un appareil destiné à mesurer la densité osseuse de parties du corps d'un patient, d'un examen de type antéro-postérieur de ce patient à un examen de type latéral, ou inversement. Dans un tel appareil, un patient est couché sur un lit d'examen, en général sur le dos. Dans une présentation antéro-postérieure, un rayonnement X produit par un tube radiogène traverse le patient du haut vers le bas, avant d'être reçu sur un détecteur de radiations situé sous le lit. Dans un examen de type latéral, le rayonnement X pénètre sur un côté du patient et ressort par l'autre côté. Les examens de type antéro-postérieur ont pour but de mesurer l'intégrité des os du bassin du patient : essentiellement le sacrum et le col du fémur. Les examens de type latéral ont pour objet de mesurer l'intégrité de sa colonne vertébrale. Pour limiter les frais, il est connu que les installations comportant de tels appareils doivent être capables de permettre les deux incidences. La mesure de la densité osseuse sert à évaluer la porosité des os et donc leur résistance aux contraintes.

La mesure de la densité osseuse est connue dans ses principes. Elle consiste à irradier avec un rayonnement d'énergie donnée le corps d'un patient ; à mesurer l'atténuation de cette irradiation ; et à recommencer l'examen pour une autre énergie donnée du rayonnement. Compte tenu de la dualité du phénomène d'absorption de l'énergie d'irradiation dans le corps, absorption Compton d'une part et absorption photoélectrique d'autre part, il est possible de différencier les tissus, du fait de ces mesures, selon les susceptibilités d'absorption dans l'un et l'autre de ces modes. En définitive, l'absorption radiologique des tissus est déterminée dans un espace à deux dimensions : une dimension selon l'effet Compton et une autre selon l'effet photoélectrique. Il est par ailleurs connu que les os sont, relativement, plus absorbant dans l'un de ces modes et que les tissus mous sont, relativement, plus absorbant dans l'autre mode. Aussi par un changement d'axe il est connu d'utiliser une de ces dimensions comme révélatrice de la densité osseuse seule, l'autre étant révélatrice de la proportion de tissu mou dans le corps. Pour exécuter de tels examens, il est donc nécessaire de disposer d'un tube à rayons X émettant à deux énergies, et d'un détecteur capable de différencier les atténuations de rayonnements à deux énergies différentes. En radiologie classique on effectue deux expériences successives dans le temps, le spectre d'émission du tube à rayons X étant modifié d'une expérience à l'autre et on déduit des mesures les densités osseuses. On présente les résultats de ces mesures dans les images.

La combinaison de ce principe de mesure avec l'utilisation de gamma-caméras est également connue. Elle conduit à une simplification des procédés en ce sens qu'une seule expérience doit seulement être tentée. En effet une gamma-caméra est capable, en elle même (dans la mesure où elle est bi-canal) de compter des coups dans une énergie donnée et dans l'autre, sans mélanger leur nombre. Le principe d'une gamma-caméra, dite aussi caméra de Anger, est le suivant. La détection des rayons gamma, après la traversée du patient, se fait à l'aide de détecteurs à scintillation. L'énergie incidente gamma est transformée en énergie lumineuse en excitant un scintillateur constitué d'un cristal d'iodure de sodium activé au thallium (NaI(Tl)). Le cristal scintillateur ainsi disposé sur le trajet des rayons gamma après leur traversée du corps examiné, transforme ces rayons gamma en rayons lumineux. Le détecteur comporte également, en aval du scintillateur, un réseau de tubes photomultiplicateurs. Chaque tube photomultiplicateur du réseau permet la transformation de l'énergie lumineuse issue du scintillateur en des impulsions électriques. L'amplitude de chaque impulsion électrique est proportionnelle à l'énergie lumineuse incidente et donc à l'énergie du photon gamma délivré au cristal.

Après amplification, les impulsions électriques sont sélectionnées en gamme d'énergie, à l'aide d'un sélecteur d'amplitude de canal. Normalement dans une gamma-caméra, on sélectionne une seule gamme ou fenêtre en amplitude. Néanmoins, il est connu, en particulier avec les appareils spécialisés de mesure de densité osseuse par gamma-caméra, d'utiliser des doubles fenêtres. Autrement dit, un photon gamma qui traverse le corps excite le cristal scintillateur, se transforme en un photon lumineux qui donne lieu, au travers du réseau des tubes photomultiplicateurs de détection, à un signal électrique dont l'amplitude est fonction de l'énergie gamma incidente. Il est donc possible de compter cette scintillation, pour l'endroit où elle s'est produite, comme un coup selon l'une des deux énergies. L'endroit où s'est produit une scintillation est déduit, par calcul de barrycentre, en traitant tous les signaux électriques produits simultanément au moment de cette scintillation par tout ou partie des tubes photomultiplicateurs du réseau. Ce traitement est généralement conduit par un ordinateur. Au bout d'une certaine durée, on est capable, pour une région donnée du scintillateur et donc en conséquence pour une région correspondante dans le corps du patient, d'établir la proportion de coups selon l'une énergie et selon l'autre énergie. Selon que cette proportion est forte ou faible à l'endroit considéré, on dira que les rayons gamma qui ont traversé le corps à cet endroit ont traversé des zones plus ou moins osseuses. Par des traitements d'images ultérieurs, il est possible ensuite de représenter la nature osseuse concernée. Ce type d'examen est très utile pour mettre en particulier en évidence les troubles d'ostéoporose.

Pour produire un rayonnement double énergie simultané, on utilise par exemple un tube à rayons X émettant un spectre sensiblement continu, par exemple entre 20 et 80 KeV, alimenté avec une tension d'anode égale sensiblement à 80 KV par rapport à la cathode. On pourrait aussi utiliser une double source chimique.En faisant ensuite passer le rayonnement émis, avant son passage au travers du corps du patient à examiner, au travers d'un filtre d'oxyde de néodyme (Nd₂O₃) ayant une densité de 0,4 gr/cm², on est capable d'obtenir un spectre biphotonique présentant deux pics d'énergie à environ 35 KeV et 43KeV. Le passage dans le filtre à pour conséquence, malheureusement, la perte de 97% des photons X émis par le tube. C'est cependant le prix à payer pour passer d'un spectre continu à un spectre biphotonique.

Il est connu, pour faire un examen du corps entier du patient, de limiter géométriquement le rayonnement X, après filtrage, à un mince faisceau plat en éventail. On déplace alors au cours de l'examen, le corps du patient perpendiculairement au plan de ce faisceau plat de manière à ce que ce faisceau balaye tout ce corps. Dans ce cas, en face du faisceau de rayonnement X en éventail on arrange le réseau des tubes photomultiplicateurs en une rangée. Les bases de ces tubes photomultiplicateurs, alignées les unes avec les autres, constituent alors un segment de détection. Plus ce segment de détection est grand, plus le nombre de tubes est grand, et plus le champ examiné est grand. Ce segment de détection est réglé minutieusement dans le plan du faisceau en éventail et à une distance prédéterminée de la source biphotonique X.

Malheureusement, pour des raisons technologiques, il n'est pas possible d'obtenir un champ de détection très grand. Dans la pratique, même avec une réalisation dans laquelle déjà 24 tubes photomultiplicateurs sont alignés les uns avec les autres, on n'aboutit qu'à un champ de l'ordre de 20 cm. Autrement dit, la partie examinée du corps est la partie d'une section de ce corps qui se trouve comprise entre le foyer du tube à rayons X d'une part et entre les 20 cm du segment de détection d'autre part. On comprend aisément que pour que cette section soit la plus grande possible, il convient de réduire au maximum les effets d'agrandissement, et donc de placer le détecteur le plus près possible du corps du patient.

Cette contrainte impose alors que, quand le patient est couché dans un lit, pendant un examen du type antéro-postérieur, le détecteur soit plaqué contre le dessous ou lit. Par contre, dans un examen du type latéral, le détecteur doit être plaqué le plus près possible du côté du patient examiné.

Dans un appareil permettant ces deux examens, le tube X et le détecteur de rayonnement sont portés par les extrémités respectives d'un arceau circulaire.

Pour passer d'un examen à l'autre on fait coulisser l'arceau circulaire mobile. On se heurte alors à des problèmes d'encombrement au moment de ce coulissement. Dans une solution de type connu, il est alors prévu, pour passer d'un examen antéro-postérieur à un examen latéral, de commencer par écarter le détecteur du dessous du lit. On déplace alors le détecteur par rapport à l'arceau et dans le plan de cet arceau. Une fois que ce déplacement est effectué, on passe d'une incidence à l'autre par coulissement de l'arceau. Puis on vient replacer le détecteur dans sa position normalisée par rapport au tube. Cette solution présente l'inconvénient que les détecteurs de type gamma-caméra sont très délicats à régler. En particulier la chaîne de traitement de localisation pour savoir où se sont produites les scintillations sur le détecteur en fonction des photomultiplicateurs l'ayant détectée, est très sensible à un déréglage, ou un recalage incorrect, du détecteur par rapport au foyer du tube à rayons X. Une telle solution n'est donc pas fiable parce que d'un examen à l'autre, pour un même patient, elle ne permet pas de comparer les images entre elles.

En outre, les deux types d'examen nécessitent des présentations différentes du patient sur le lit. Pour un examen de type antéro-postérieur, il est nécessaire de déplacer horizontalement en correspondance le tube à rayons X et le détecteur, longitudinalement le long du patient, ou transversalement à ce dernier. Par contre, pour l'examen de type latéral, la cinématique de basculement par coulissement est telle que la colonne vertébrale (à examiner) du patient se trouve alors bien en-dessous de l'alignement segment de détection-foyer du tube à rayons X, même si le segment de détection est alors vertical. Pour résoudre ce problème il est connu de demander alors au patient de se lever du lit d'examen et de placer sur celui-ci un matelas plus épais de manière à faire remonter verticalement sa colonne vertébrale. Outre la manipulation de ce matelas supplémentaire, l'allongement ainsi que le relèvement du patient sont des manipulations qui font perdre du temps et donc qui rendent l'appareil peu rentable. Dans une variante, le basculement n'est pas complet. L'examen n'est alors pas tout à fait latéral. Le tube à rayons X tire en biais vers le bas sur le détecteur. On peut montrer que dans ce cas l'examen n'est pas très académique mais qu'en plus du fait de la forme du lit le détecteur se trouve alors loin du patient, ce qui réduit la section utile d'examen.

La description du document WO-A-8 808 688 prévoit un déplacement latéral (figure 3) d'un ensemble comportant un détecteur (14) un arceau portique (12) et une source (21), puis la rotation de cet ensemble autour d'un axe (18). Cependant, comme la description de ce brevet l'indique, cette solution présente l'inconvénient de ne pas être une solution équilibrée. Elle nécessite au contraire d'additionner des poids (21) sur le détecteur pour ajuster le centre de gravité dans le plan vertical. L'avantage de l'invention se situe, vis à vis de ce document, par le remplacement de l'axe par un arceau coulissant dans une coulisse portée par un socle. Ceci assure une permanence de l'équilibre de la répartition des poids, et donc une bien meilleure stabilité du dispositif que celui décrit dans ce document.

Le document EP-A-O 253 742 prévoit le passage d'une incidence de type antéropostérieure à une incidence latérale. Mais dans ce cas, il existe une désolidarisation de la liaison du détecteur à l'émetteur de rayons X, puisque chacun d'eux est fixé à un mécanisme différent. Par ailleurs, il est douteux que le mécanisme complexe décrit dans ce brevet puisse fonctionner, puisque chacun d'eux est un mécanisme de rotation autour d'axes qui d'autre part sont perpendiculaires l'un à l'autre. On voit mal comment cette solution pourrait être facilement réglée. Un des problèmes résolus avec l'invention est justement d'éviter le déréglage de l'alignement du tube à rayons X sur le détecteur.

L'invention à pour objet de remédier aux inconvénients des appareils connus, en gardant essentiellement le détecteur en position fixe par rapport au tube à rayons X. Ce détecteur et ce tube sont alors définitivement montés et fixés de part et d'autre d'un arceau qui peut coulisser.

Plutôt que de provoquer, au moment du changement de type d'examen, l'éloignement du détecteur de l'arceau, on provoque alors le déplacement en translation de la totalité de l'arceau. Cette translation vient en butée, d'une part à une extrémité du lit (vers les pieds de manière à ne prendre aucun risque vis-à-vis du patient), et d'autre part en translation latérale dans le plan de l'arceau. Ce faisant, on utilise pour effectuer le basculement, des possibilités déjà existantes de déplacement de l'appareil, sans avoir à utiliser une autre possibilité de déplacement et donc à mettre en oeuvre un décalage du détecteur, dont par ailleurs on a indiqué qu'il était néfaste.

L'invention a donc pour objet un procédé de basculement, avec un appareil destiné à mesurer la densité osseuse de parties du corps d'un patient, d'un examen de type antéro-postérieur de ce patient à un examen de type latéral de ce patient, cet appareil comportant un châssis, ce châssis portant un lit et un dispositif de mesure, ce dispositif de mesure étant muni d'un tube radiogène maintenu, relativement au châssis et en vis-à-vis d'un détecteur de rayonnements, par un arceau de basculement circulaire et mobile en coulissement, caractérisé en ce qu'il comporte les phases suivantes :
- on place le dispositif de mesure de cet appareil en butée à une extrémité longitudinale du lit,
- on déplace par translation de la totalité de l'arceau dans son propre plan, le dispositif de mesure par rapport au lit,
- on effectue le basculement en faisant coulisser l'arceau dans une coulisse fixée au châssis,
- on déplace à nouveau par translation de la totalité de l'arceau dans son propre plan mais en sens contraire cette fois, le dispositif de mesure pour lui faire occuper une position correspondant à l'examen à entreprendre, le tube radiogène étant toujours maintenu fixe par rapport au détecteur de rayonnements.

La présente invention sera mieux comprise à la lecture de la description qui suit et à l'examen des figures oui l'accompagnent. Celles-ci ne sont données qu'à titre indicatif et nullement limitatif de l'invention. Les figures montrent :
- figure 1 : une représentation schématique d'une vue en coupe de l'appareil selon l'invention ;
- figures 2 et 3 : des représentations physiques de l'appareil de l'invention dans des incidences antéro-postérieure et latérale respectivement. La figure 3 montre par ailleurs le plan de coupe dans lequel est représentée la figure 1.

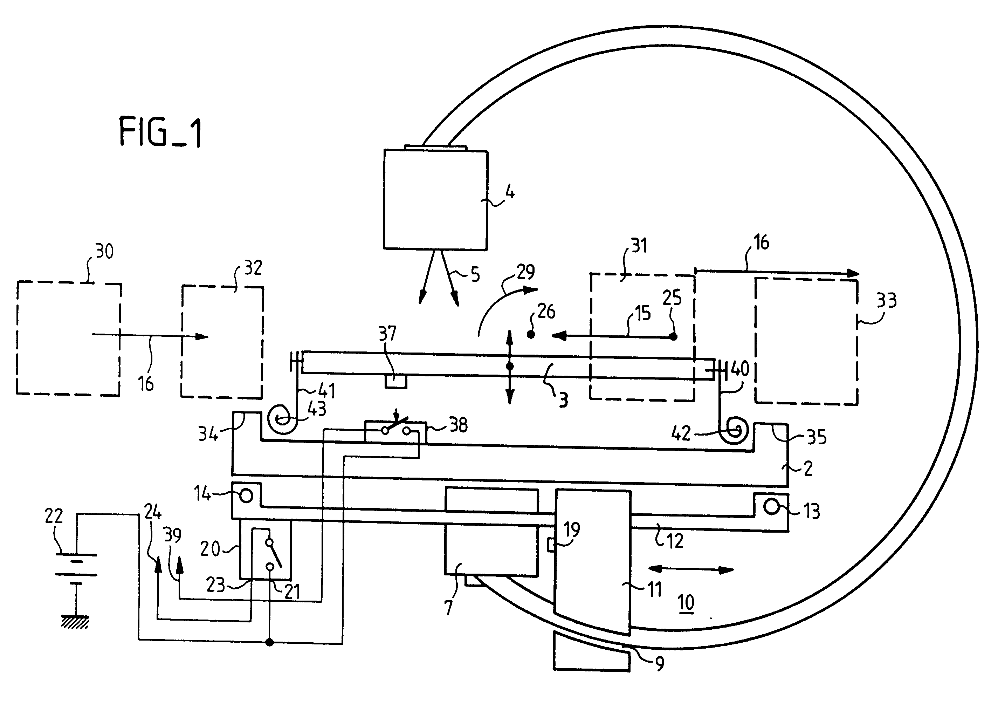

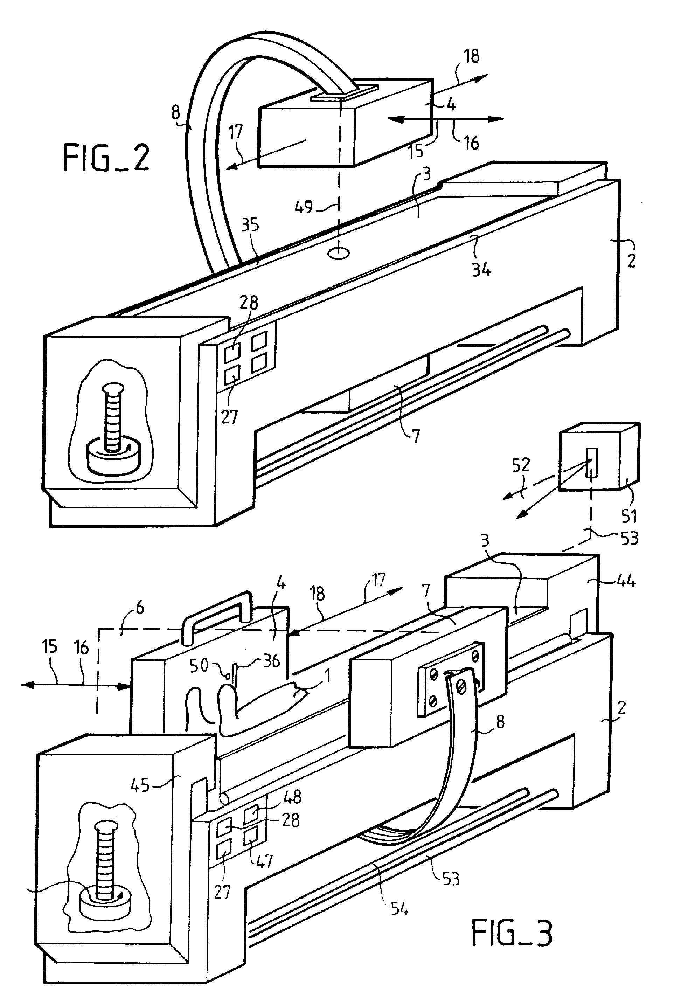

Les figures montrent, avec les mêmes références, un appareil destiné à mesurer la densité osseuse de partie du corps d'un patient 1. L'appareil comporte un châssis 2 qui porte un lit 3 et un dispositif de mesure. Le châssis 2 est par ailleurs muni de deux entretoises 53 et 54 de raidissement. Le lit 3 est en un matériau transparent aux rayons X. Le dispositif de mesure comporte un tube radiogène 4 émettant un faisceau plat en éventail de rayonnement X 5. Ce faisceau plat en éventail se déploie dans un plan tel que le plan 6 (plan de coupe sur la figure 3 de la Figure 1) perpendiculaire à la grande longueur du lit 3 sur lequel est allongé le patient. En face du tube radiogène 4 est disposé un détecteur de radiations 7. Le détecteur de radiations 7 est un détecteur du type de ceux utilisé dans les gamma-caméras. Il comporte, de l'amont à l'aval du rayonnement, un scintillateur et un alignement de tubes photomultiplicateurs dont les bases de détection forment un segment de détection contenu dans le plan 6. Le détecteur 7 est par ailleurs relié à des dispositifs de traitement de signal et de visualisation d'images, de type connu, et non représentés ici. Le tube radiogène 4 et le détecteur de rayonnement 7 sont fixés l'un à l'autre par l'intermédiaire d'un arceau circulaire 8. L'arceau 8 dans l'exemple est légèrement plus grand qu'un demi-cercle de sorte que le tube 4 et le détecteur 7 sont placés de l'autre côté du centre de rotation de cet arceau par rapport à l'arceau lui-même.

L'arceau circulaire est mobile et peut coulisser dans une coulisse 9 solidaire d'un équipage mobile 10. L'équipage mobile 10 comporte essentiellement un socle 11 dans lequel par exemple peut être gravée fixement la coulisse 9. L'équipage 10 comporte aussi un jeu de roulement à billes (non représenté) pour permettre au socle 11 de coulisser lui-même en translation, perpendiculairement au sens allongé du lit, sur un portique 12. Le portique 12 est montré ici avec un seul rail, mais dans la pratique il y en a deux. Un premier rail est proche de l'observateur qui regarde la figure 1. Un deuxième rail est éloigné de cet observateur. Les deux rails permettent la stabilité de l'arceau 8. Les deux rails sont décalés du plan 6 dans lequel se trouve le détecteur 7 de manière à ce que celui-ci ne vienne pas les rencontrer. Le portique 12 lui-même est déplaçable en translation, longitudinalement au lit, par coulissement le long de deux arbres 13 et 14. Les deux arbres 13 et 14 parallèles au lit sont reliés chacun à leurs extrémités au châssis 2. Alors que le portique 12 autorise une translation du dispositif de mesure dans le sens des flèches 15 et 16, les arbres 13 et 14 autorisent le déplacement de l'équipage mobile 10 (et donc de tout le dispositif de mesure) dans le sens des flèches 17 et 18 (figures 2 et 3 seulement).

Le procédé de l'invention est le suivant. Une fois que le patient est sur le lit 3, et qu'on veut passer de l'examen antéro-postérieur à l'examen latéral, on place le dispositif de mesure 4, 7, 8 longitudinalement en butée à une extrémité de ce lit. Par exemple, de préférence, on le place du côté des pieds du patient, de manière à ne prendre aucun risque de blesser ce dernier au moment de la manoeuvre. Dans la pratique, le lit peut même être suffisamment long pour que, pour la plupart des patients, le dispositif de mesure vienne même à l'aplomb au-delà des pieds de ces patients. Ceci étant exécuté, on déplace transversalement, dans le sens de la flèche 15, le dispositif de mesure 4, 7, 11, en faisant coulisser le socle 11 le long du portique 12. On effectue ce déplacement jusqu'à ce qu'un taquet 19, solidaire du socle 11, vienne fermer en butée un interrupteur de type fin de course 20.

L'interrupteur 20 comporte par exemple deux bornes de signaux électriques, une première borne 21 est reliée à une alimentation 22, et une deuxième borne 23 sert à délivrer un signal de validation 24. Un signal de validation 24 est utilisé dans un système logique de commande de manipulation de l'appareil pour autoriser ensuite la rotation du dispositif de mesure.

Au moment de cette translation transversale, le centre de rotation du dispositif de mesure 4, 7, 8 s'est déplacé de l'emplacement 25 à l'emplacement 26 par rapport au lit 3 (figure 1). La rotation est une rotation motorisée : le socle 11 comporte des moteurs (non représentés). Ces moteurs sont actionnés par des boutons de commande 27 et 28 visibles sur le côté du châssis 2 (figures 2 et 3). Cette rotation est de préférence motorisée car, par la motorisation électrique, il est possible de tenir compte d'un signal de validation électrique 24. Si le signal électrique 24 (sécurité positive) est présent, les boutons 27 ou 28 sont actifs. Ils sont inactifs dans le cas contraire.

Au moment où la rotation pour passer d'une incidence antéro-postérieur à une incidence latérale est ainsi terminée, rotation 29, le détecteur de rayonnement 7 le tube radiogène 4 se trouvent respectivement dans les positions 30 et 31 sur la figure 1. On constate que le tube 31 empiète alors un peu au-dessus de la position abaissée du lit 3. Par un mouvement de translation, dans le plan de l'arceau 8, et le long du portique 12, on déplace l'ensemble de mesure 10 transversalement dans l'autre sens cette fois de manière à ce que le détecteur et le tube viennent occuper les positions respectivement 32 et 33. Dans ces positions 32 et 33, le détecteur et le tube sont sensiblement au-dessus, tous les deux, du châssis 2 de part et d'autre du lit. L'écart est très faible cependant : ils ne se trouvent que 2 cm au-dessus.

Le châssis 2 a ainsi la forme d'une cuvette pouvant recevoir le lit 3 dans son creux. Cette cuvette est entourée de bords longitudinaux 34 et 35 au-dessus et à l'aplomb desquels le détecteur et le tube peuvent se déplacer en translation, par coulissement de l'ensemble 10 le long des rails 13 et 14.

La figure 1 montre la fonctionnalité supplémentaire du lit 3 par rapport au châssis 2 : le lit peut être élevé. Sur la figure 2, le lit est dans la position basse : on effectue un examen de type antéro-postérieur, le corps du patient est alors amené le plus près possible du détecteur 7 qui se trouve par en-dessous. Le dessus du lit affleure le dessus des bords 34 et 35. Par contre, sur la figure 3, compte tenu de la position de la colonne vertébrale de ce patient, le lit a été élevé de manière à ce que la colonne vertébrale de ce patient soit située sensiblement au milieu du champ irradié par le rayonnement du tube 4. Le champ irradié est un champ plat passant par le diaphragme allongé 36 du tube 4. Comme la figure 1 le montre, dans la réalisation préférée où le lit peut être soulevé, il est nécessaire pour effectuer le basculement, que le lit soit dans une position abaissée, de manière à ce que le tube à rayons X 4 puisse effectivement occuper l'emplacement 31. Dans ce but, le lit qui est manoeuvré par ailleurs est solidaire d'un taquet 37 destiné à fermer, quand le lit est abaissé, un interrupteur de type fin de course 38, lui aussi relié à l'alimentation 22 et délivrant un signal de validation 39. En pratique, dans la variante préférée, le mouvement de rotation par les commandes 27 et 28 n'est autorisé que si les deux signaux 24 et 39 sont actifs. Pour tenir compte des différentes tailles des patients, l'élèvement du lit peut être de l'ordre de 20 cm. Ainsi dans l'invention, pour basculer d'un examen à l'autre, on se met en butée au pied du lit. On se déplace transversalement. On effectue la rotation. On se redéplace transversalement dans l'autre sens. Et enfin, on relève le lit. Cette série de manipulations, dont la séquence pourrait d'ailleurs être automatique, remplacent les opérations de désaccouplement du détecteur 7 et du tube 4.

Pour éviter, au moment où le lit s'abaisse, que le patient par inadvertance vienne à se laisser coincer les mains entre le lit et le creux du châssis 2, les bords du lit sont munis de deux rideaux 40 et 41, enroulés de manière élastique sur des mâts respectivement 42 et 43, et fixés par ailleurs à leur sommet par des rivets sur les chants du lit 3. Lorsque le lit s'élève les rideaux se déploient, lorsque le lit s'abaisse, par réaction élastique ils se rembobinent sur les mâts 42 et 43. Pour permettre l'élèvement du lit, le lit est porté à ses deux extrémités longitudinales par des potences 44 et 45 dont les mâts verticaux peuvent s'élever le long du châssis 2 sous l'action de moteurs tels que 46 déclenchés par des boutons de commande 47 ou 48 situés à proximité des boutons 27, 28.

Dans les appareils de type connu, le déplacement selon les flèches 15 à 18 était connu. Dans l'invention néanmoins, on a augmenté l'amplitude de ce déplacement de manière à permettre au détecteur de rayonnement 7 d'échapper du bord 34 du châssis 2 de manière à remonter pour occuper la position 30. Une fois qu'il a occupé cette position 30, on ramène l'arceau dans la position correspondant à 32 et 33. Par un dispositif de type fin de course analogue à ceux qui ont été vus jusqu'à présent, on peut n'autoriser le fonctionnement du dispositif de mesure (la mesure proprement dite) que si le tube et le détecteur occupe les positions 32 et 33. Avantageusement, l'occupation de ces positions 32 et 33 correspond au maximum de déplacement vers la droite du dispositif de mesure.

En examen de type antéro-postérieur, pour régler la position du dispositif de mesure par rapport au patient, on utilise une source lumineuse, par exemple une diode laser embaguée dans le tube radiogène, laissant passer un rayon lumineux 49 dont la trace permet de visualiser, sur le patient, la région qui correspondra, après traitement des images, au centre de l'image. Cette trace est pointée par construction sur le centre du segment détecteur. Le rayon lumineux 49 sort par un orifice 50 situé à proximité du diaphragme de rayonnement 36. Par contre, pour pratiquer l'examen de type latéral, on a disposé une source lumineuse 51 sur un des murs de la salle d'examen dans laquelle se trouve placé l'appareil. Par construction, la source lumineuse 51 émet un rayonnement lumineux 52 situé dans un plan 53 de préférence vertical, passant longitudinalement par le centre du lit. Compte tenu des positions fixes 32 et 33 d'autorisation de la mesure dans l'examen latéral, le rayonnement lumineux 52 permet de placer le patient le mieux possible sur le lit 3. Dans ce cas, celui-ci se déplace lui-même par reptation en fonction des indications que lui donne un opérateur qui visualise la trace sur le corps de ce patient du rayonnement lumineux 52.

Les opérations sont faites en sens inverse lorsqu'on passe de l'examen latéral à l'autre examen.

## Revendications

1. Procédé de basculement, avec un appareil destiné à mesurer la densité osseuse de parties du corps d'un patient (1), d'un examen de type antéro-postérieur de ce patient à un examen de type latéral de ce patient, cet appareil comportant un châssis (2), ce châssis portant un lit (3) et un dispositif de mesure, ce dispositif de mesure étant muni d'un tube radiogène (4) maintenu, relativement au châssis et en vis à vis d'un détecteur (7) de rayonnements (5), par un arceau (8) de basculement circulaire et mobile en coulissement (9), caractérisé en ce qu'il comporte les phases suivantes:
- on place le dispositif de mesure de cet appareil en butée à une extrémité longitudinale du lit,
- on déplace (15) par translation (12) de la totalité de l'arceau dans son propre plan, le dispositif de mesure par rapport au lit,
- on effectue le basculement (29) en faisant coulisser l'arceau dans une coulisse fixée au châssis,
- on déplace à nouveau (16) par translation de la totalité de l'arceau dans son propre plan mais en sens contraire cette fois, le dispositif de mesure pour lui faire occuper une position (32, 33) correspondant à l'examen à entreprendre, le tube radiogène étant toujours maintenu fixe par rapport au détecteur de rayonnements.

2. Procédé selon la revendication 1, caractérisé en ce qu'on n'autorise le basculement que pour une position en butée, à la fois à proximité des pieds du patient et en limite (20) de déplacement en translation transversale dans le plan de l'arceau, du dispositif de mesure.

3. Procédé selon l'une quelconque des revendications 1 ou 2, caractérisé en ce que pour effectuer l'examen de type latéral on n'autorise le fonctionnement du dispositif de mesure que pour une position (32, 33) dite d'examen de ce dispositif, cette position étant différente en translation dans le plan de l'arceau de la position (30, 31) en butée pour laquelle on peut effectuer le basculement.

4. Procédé selon l'une quelconque des revendications 1 à 3, caractérisé en ce que pour effectuer l'examen de type latéral on soulève (44-46) le lit par rapport au châssis de manière à présenter la colonne vertébrale du patient au mieux dans le champ du détecteur de rayonnements, mais en ce qu'on n'autorise par contre le basculement que pour une position abaissée (38) en butée du lit.

5. Procédé selon la revendication 4, caractérisé en ce que pour soulever le lit on déploie (40-43) entre le lit et le châssis des rideaux enroulés pour éviter que le patient ne puisse se coincer les mains entre ce lit et ce châssis.

6. Procédé selon l'une quelconque des revendications 1 à 5, caractérisé en ce que pour déplacer le dispositif de mesure dans le plan de l'arceau et pour régler la position de ce dispositif de mesure par rapport au patient on se sert d'une indication lumineuse émise par une source lumineuse (50) embarqué dans le tube radiogène (4) et pointée par construction en direction du centre du détecteur.

7. Procédé selon l'une quelconque des revendications 1 à 6, caractérisé en ce que pour l'examen de type latéral on règle la position du corps du patient par rapport au lit en se servant d'une indication lumineuse (51-53) produite par une source lumineuse placée à l'extérieur de l'appareil et pointant par construction au moment du montage, sur le milieu du lit.

## Patentansprüche

1. Mit einem Gerät zum Messen der Knochendichte von Teilen des Körpers eines Patienten (1) durchfürbares Verfahren zum Umschwenken von einer Untersuchung dieses Patienten von vorne nach hinten auf eine Untersuchung des Patienten in seitlicher Richtung, wobei das Gerät ein Untergestell (2) aufweist, das ein Bett (3) und eine Meßvorrichtung trägt, die mit einer Röntgenröhre (4) versehen ist, die mit Hilfe eines Kreisschwenkbogens (8) in einer Gleitführung (9) beweglich relativ zu dem Untergestell und gegenüber einem Detektor (7) für Strählungen (5) gehalten ist, **dadurch gekennzeichnet,** daß es folgende Phasen aufweist:
- die Meßvorrichtung dieses Geräts wird an einem Tängsende des Betts in Anlage gebracht,
- durch Translation (12) des gesamten Bogens in seiner Ebene wird die Meßvorrichtung in bezug auf das Bett verschoben (15),
- das Umschwenken (29) wird durchgeführt, indem der Bogen in einer am Untergestell befestigten Gleitführung verschoben wird,
- durch Translation des gesamten Bogens in seiner Ebene, jedoch dieses Mal im entgegengesetzten Sinn, wird die Meßvorrichtung erneut verschoben (16), damit sie eine Position (32, 33) entsprechend der durchführenden Untersuchung einnimmt, wobei die Röntgenröhre immer noch bezüglich des Strählungsdetektors festgehalten ist.

2. Verfahren nach Anspruch 1, **dadurch gekennzeichnet,** daß das Umschwenken nur in einer Anschlagposition nahe den Füßen des Patienten und an einer Grenze (20) der Quertranslationsverschiebung in der Ebene des Bogens der Meßvorrichtung freigegeben wird.

3. Verfahren nach einem der Ansprüche 1 oder 2, **dadurch gekennzeichnet,** daß zur Durchführung der Untersuchung des seitlichen Typs der Betrieb der Meßvorrichtung nur für eine als Untersuchungsposition dieser Vorrichtung bezeichnete Position (32, 33) freigegeben wird, wobei diese Position hinsichtlich der Translation in der Ebene des Bogens von der Anschlagposition (30, 31) verschieden ist, in der das Umschwenken durchgeführt wird.

4. Verfahren nach einem der Ansprüche 1 bis 3, **dadurch gekennzeichnet,** daß zur Durchführung der Untersuchung des seitlichen Typs das Bett bezüglich des Untergestells so angehoben wird (44 bis 46), daß die Wirbelsäule des Patienten am besten im Feld des Strahlungsdetektors liegt, und daß das Umschwenken jedoch im Gegensatz dazu nur bei einer abgesenkten Anschlagposition (38) des Betts freigegeben wird.

5. Verfahren nach Anspruch 4, **dadurch gekennzeichnet,** daß zum Anheben des Betts zwischen dem Bett und dem Untergestell aufgewickelte Vorhänge (40 bis 43) abgewickelt werden, damit verhindert wird, daß sich der Patient die Hände zwischen dem Bett und dem Untergestell einklemmt.

6. Verfahren nach einem der Ansprüche 1 bis 5, **dadurch gekennzeichnet,** daß zum Verschieben der Meßvorrichtung in der Ebene des Bogens und zum Einstellen der Position der Meßvorrichtung bezüglich des Patienten von einer Leuchtanzeige Gebrauch gemacht wird, die von einer Lichtquelle (50) abgestrahlt wird, die in der Röntgenröhre (4) untergebracht ist und konstruktionsbedingt in Richtung der Mitte des Detektors zeigt.

7. Verfahren nach einem der Ansprüche 1 bis 6, **dadurch gekennzeichnet,** daß zur Untersuchung vom seitlichen Typ die Position des Körpers des Patienten bezüglich des Betts dadurch eingestellt wird, daß von einer Leuchtanzeige (51 bis 53) Gebrauch gemacht wird, die von einer Lichtquelle erzeugt wird, die außerhalb des Geräts angebracht ist und konstruktionsbedingt im Zeitpunkt der Montage auf die Mitte des Bettes gerichtet ist.

## Claims

1. Tilting process, with an apparatus designed to measure the osteodensity of parts of the body of a patient (1), from an examination of the anterior-posterior type to an examination of the lateral type of this patient, this apparatus comprising a frame (2), this frame bearing a bed (3) and a measuring device, this measuring device being fitted with a radiogenic tube (4) secured in position in relation to the frame and opposite a detector (7) of rays (5), by an arch (8) performing a circular tilting movement and slidable (9), characterised by the fact that it comprises the following phases:
- the measuring device of this apparatus is positioned to abut against one longitudinal end of the bed,
- the measuring device is displaced (15) in respect of the bed by traversing (12) the entire arch in its own plane,
- the tilting (29) is effected by sliding the arch in a slide affixed to the frame,
- by the traversing of the entire arch in its own plane but this time in the opposite direction, the measuring device is once again displaced (16) in order to cause it to assume a position (32, 33) corresponding to the examination to be effected, the radiogenic tube being still secured in a fixed position in relation to the ray detector.

2. Process in accordance with claim 1, characterised by the fact that the tilting is only rendered possible for an abutting position, both near the patient's feet and at the limit (20) of the transversal traversing movement in the plane of the arch, of the measuring device.

3. Process in accordance with either one of claims 1 or 2, characterised by the fact that in order to effect the examination of the lateral type the operation of the measuring device is only rendered possible for a position (32, 33) termed the examination position of this device, this position being different, as regards the traversing in the plane of the arch, from the abutting position (30, 31) for which the tilting can be effected.

4. Process in accordance with any one of claims 1 to 3, characterised by the fact that in order to effect the examination of the lateral type the bed is raised (44-46) in relation to the frame in order to position the patient's spinal column in the field of the ray detector as satisfactorily as possible but that the titlting on the other hand is only rendered possible for a lowered (38) abutting position of the bed.

5. Process in accordance with claim 4, characterised by the fact that in order to raise the bed rolled curtains are spread (40-43) between the bed and the frame in order to prevent the patient from getting his hands jammed between the bed and the frame.

6. Process in accordance with any one of claims 1 to 5, characterised by the fact that in order to displace the measuring device in the plane of the arch and to regulate the position of this measuring device in relation to the patient a light signal is used which is emitted by a light source (50) inserted in the radiogenic tube (4) and designed to point towards the centre of the detector.

7. Process in accordance with any one of claims 1 to 6, characterised by the fact that for the examination of the lateral type the position of the patient's body in relation to the bed is regulated by means of a light signal (51-53) produced by a light source mounted outside the apparatus and designed to point, when it is mounted, towards the middle of the bed.
